# EUROPEAN PATENT APPLICATION

(11) **EP 3 944 251 A1**
(43) Date of publication of application: **26.01.2022**
(21) Application number: 20187431.0
(22) Date of filing: 23.07.2020
(51) Int. Cl.: G16H 20/10, G16H 50/70, G16H 10/60

(54) **WORKFLOW FOR OPTIMIZED WAKE UP PROCEDURE**

(71) Applicant: Koninklijke Philips N.V., 5656 AG Eindhoven (NL)
(72) Inventor: VOGTMEIER, Gereon, 5656 AE Eindhoven (NL); BUSSA, Nagaraju, 5656 AE Eindhoven (NL); WEISS, Steffen, 5656 AE Eindhoven (NL); JOHNSON, Mark, 5656 AE Eindhoven (NL); LEUSSLER, Christoph, 5656 AE Eindhoven (NL)
(74) Representative: Philips Intellectual Property & Standards

(57) **Abstract**

The present invention relates to wake-up management. An apparatus is provided for assessing a wake-up procedure of a sedated patient in an imaging process. The apparatus comprises an input unit, a processing unit, and an output unit. The input unit is configured to receive patient profile data and sedation data comprising information about a sedation state of the sedated patient. The processing unit is configured to apply a data-driven model to the patient profile data and sedation data of the sedated patient to estimate at least one timing in the wake-up procedure of the sedated patient. The data-driven model has been trained on a training dataset obtained from historical data of one or more patients. The training dataset comprises patient profile data and sedation data of the one or more patients as training inputs and at least one timing in the wake-up procedure of the one or more patients as training outputs. The output unit is configured to provide the at least one estimated timing.

The patient's reaction to the sedation medication is patient specific and also the timing of the wake up procedure is patient specific. The timing prediction may help in understanding when the patient will wake-up from the sedation and hence next steps in the workflow, such as post imaging as exiting the bore, and/or performing autonomous standing up from the patient support.

## Description

### FIELD OF THE INVENTION

The present invention relates to an apparatus for assessing a wake-up procedure of a sedated patient in an imaging process, to a wake-up management system, to a method of assessing a wake-up procedure of a sedated patient in an imaging process, and to a computer program element.

### BACKGROUND OF THE INVENTION

High patient throughput is crucial for many medical imaging facilities. At the same time, patient engagement and experience are becoming more important and are even part of the reimbursement in selected markets like the US. Moreover, imaging will become more and more autonomous in future with less operator depended actions and automated workflow steps, for increasing the throughput while reducing the operational costs. Less operator dependency also gives the objectivity in the imaging procedures and helps in personalizing to the patient.

Decreased patient throughput can be a consequence of multiple different issues within the workflow, such as delayed patient show-up, unexpected patient behavior (e.g. due to lack of information, anxiety, etc.), patients unable to follow instructions (e.g. breath holds, lying still etc.). Moreover, image degradation due to artefacts and repeated measurements or even revisits of patients may be often caused by improper patient information so that workflow changes or imaging protocol adaptions have to be made on the fly by the operator. This may add more tasks to the workload of operators despite the fact that not every operator is trained sufficiently to handle such situations, to pick the right sequence and adapt the parameters in the most optimized way.

One of the critical workflow aspects may be the wake up procedure after the sedation and the imaging, especially in the case of autonomous imaging where either the patient has to be active or technical support replaces staff.

### SUMMARY OF THE INVENTION

There may be a need to improve wake-up management.

The object of the present invention is solved by the subject-matter of the independent claims, wherein further embodiments are incorporated in the dependent claims. It should be noted that the following described aspects of the invention apply also for the apparatus for assessing a wake-up procedure of a sedated patient in an imaging process, for the wake-up management system, for the method of assessing a wake-up procedure of a sedated patient in an imaging process, and for the computer program element.

According to a first aspect of the present invention, there is provided an apparatus for assessing a wake-up procedure of a sedated patient in an imaging process. The apparatus comprises an input unit, a processing unit, and an output unit. The input unit is configured to receive patient profile data and sedation data comprising information about a sedation state of the sedated patient. The processing unit is configured to apply a data-driven model to the patient profile data and sedation data of the sedated patient to estimate at least one timing in the wake-up procedure of the sedated patient. The data-driven model has been trained on a training dataset obtained from historical data of one or more patients. The training dataset comprises patient profile data and sedation data of the one or more patients as training inputs and at least one timing in the wake-up procedure of the one or more patients as training outputs. The output unit is configured to provide the at least one estimated timing.

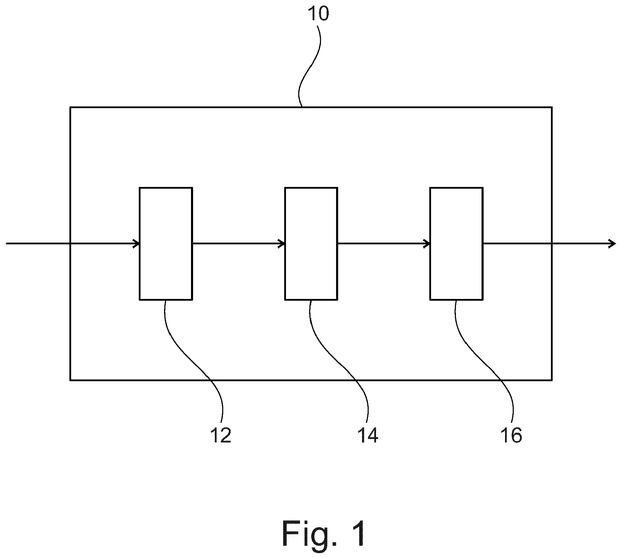

The patient profile generally constitutes the gender, age, Body Mass Index (BMI), etc. The sedation state may be derived from the sedation dose and the sedative medication used for the sedated patient. The sedation state may also be obtained by using automatic monitoring of sedation depth with e.g. a sedation tracker. Various examples of the sedation tracker will be discussed hereafter and in particular with respect to the embodiment in Fig. 1. While training the data-driven model these patient-specific parameters (i.e. patient profile) are used to calibrate the effect of certain dose of sedation on the person. In this way, one component of a data-driven model for predicting the effect (in terms of time) of the sedation on a certain individual is developed. Another component of the data-driven model works on the time series responses of the individual for the inputs for estimating the sedation levels.

Once the data-driven model is trained on the various population data and/or patient-specific data of their responses to the sedation, profile specific model can be generated. The patient's reaction to the sedation medication is patient specific and also the timing of the wake up procedure is patient specific. The patient-specific timing prediction may help in understanding when the patient will wake-up from the sedation and hence next steps in the workflow, and post imaging as exiting the bore. For example, the data-driven model may be used to estimate the time left for the patient to come out of the sedation. Based on this estimation, the workflow steps are optimized based on the prescribed scan procedures, such as speeding the scan procedures, increasing the sedation dose, etc. For example, the data-driven model may be used to estimate the time when the patient can leave the scanner in a safe mode (i.e. prediction of sedation end). Based on this estimation, autonomous standing up from the patient support can be performed in a safe way.

According to an embodiment of the present invention, the processing unit is configured to determine at least one of the following parameters based on the at least one estimated timing:
- sequence of workflow steps in the imaging process;
- imaging protocol;
- delivery of required support for the sedated patient; and
- delivery of staff support; and
wherein the output unit is configured to provide at least one determined parameter.

This workflow estimation allows a precise timing prediction for a defined wake-up procedure, which again enables a much better planning for the room and the imaging process with well adapted processes for the patient according to his individual profile. Besides higher acceptance, the method may reduce the risks as the wake up process is well controlled and improves the overall quality and patient experience.

The workflow may be optimized timewise but also with optimized patient experience and minimized risk. Based on this estimation, the workflow steps may be optimized based on the prescribed scan procedures (e.g. speeding the scan procedures, increasing the sedation dose, etc.). For example, depending on the individual patient profile also sound, music, light, and/or special smelling substances or fragrances may be used to increase the speed of the wake-up procedure. The estimated workflow may also indicate how and when the patient comes from the sedated level to a level when the patient can do next steps on his own and finally also leave the scanning room on his own. The required support for the patient, such as audio information, video guidance, transport support (e.g. automatic wheelchair), and also staff support may be estimated and delivered in time and patient specific.

According to an embodiment of the present invention, the sedation data further comprises a sedative medication and/or sedation dose used for the sedated patient.

The inclusion of the sedative medication and/or sedation dose used for the sedated patient may allow a more precise timing estimation.

According to an embodiment of the present invention, the training dataset is obtained from historical data of the sedated patient and/or from historical data of a plurality of other patients.

For patients who have been sedated (and optionally scanned) before it may be advantageous if the data considered for the data-driven model may be biased towards the previous data of the same patient i.e. the sedation history and wake-up times recorded at previous occasions

For patients who have not been sedated/scanned before, it could have the timing predicted by comparison to population history and then the sedation tracking may be followed.

According to an embodiment of the present invention the at least one timing in the wake-up procedure comprises one or more of:
- wake-up time;
- time to administer medication;
- bore-exit time; and
- stand-up time.

In an example, these wake-up prediction models are used to estimate the time left for the patient to come out of the sedation.

In an example, the time to administer medication may be estimated. For example, the timing for the injection of sedation medicine (and optionally the increase or decrease of the dose) and also the counterpart to use a kind of "wake up medication" supporting the natural human process - also some emergency wake up medication, may be estimated. A switch from "sedation" to "wake up" with different dose levels allows individual patient treatment from entering the scanner during the complete scanning process until full wake up. This may allow also the best matching trigger for imaging during the complete sequence as it is synchronized with the medication.

In an example, the bore-exit time may be estimated, which may be used to shorten the bore time.

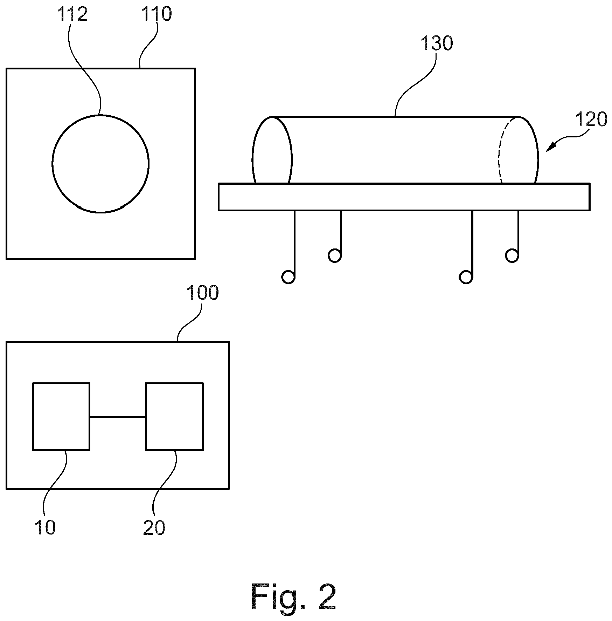

In an example, the time is estimated when the patient can leave the scanner in a safe mode, i.e. when autonomous standing up from the patient support may be performed. The most simple solution to de-risk the unsupervised wake-up of the patient would be to leave the patient in the imaging system in this phase until he is ready to be transported autonomously. However, with certain safety devices as explained hereafter and particularly with respect to the embodiment shown in Fig. 2, the patient may be moved out of the bore irrespective of sedation, which therefore shortens the bore time.

According to an embodiment of the present invention, the input unit is further configured to receive real-time measurement data indicative of a sedation state of the sedated patient. The processing unit is configured to continuously adjust the at least one estimated timing and the at least one determined parameter according to the sedation level of the sedated patient.

In this embodiment, patients could be provided with a "sedation tracker" - which is in one embodiment is any of the known sleep trackers. Further examples of the sedation tracker will be discussed hereafter and particularly with respect to Fig. 1. The output of the sedation tracker may be used to dynamically adjust the time to wake-up. With the sedation tracker, for all measurements and actions a precise time measurement may be done continuously (e.g. every 1 min, 5 min, 10 min, or 20 min) to check if the timing is as expected and would fit to the estimated timing - otherwise correction means have to be applied. The iterative approach ensures high prediction quality.

According to a second aspect of the invention, there is provided a wake-up management system. The wake-up management system comprises:
- an apparatus according to the first aspect of the invention and any associated example; and
- a controller configured to generate a trigger signal for the sedated patient and/or one or more devices to perform an action based on the at least one estimated timing in the wake-up procedure.

In other words, the wake-up management system may trigger signals for the patient to receive sedation dose, enter the bore, leave the bore, retract the extra bore wall/fence of the mobile patient support, control the content of the patient entertainment system, and/or allow the patient to stand up from the patient support. In this way, the workflow is optimized timewise but also with optimized patient experience and minimized risk. For example, the risk of any uncontrolled action by the patient may be minimized with the extra bore wall/fence of the mobile patient support and/or the patient entertainment system, so that there is less or no risk even in case there is no staff available directly or within a short period of time. This may also allow to reduce the bore-time, because the patient may be moved out of the bore irrespective of sedation. This will be explained hereafter and in particular with respect to the embodiment shown in Fig. 2.

According to an embodiment of the present invention, the one or more devices comprise a mobile patient support for transferring a patient to and from a medical imaging system. The mobile patient support comprises a safety device for preventing the sedated patient from falling down from the mobile patient support during transport.

Moving a sedated patient out of the bore autonomously on a self-driving patient support may confuse the patient considerably and induce the risk of anxiety or even falling down from the support. This embodiment provides means for a safe and undisturbed unsupervised exit of the patient from the bore under sedation. Similarly, the embodiment also enables to induce sedation before the patient enters the imaging system, which again shortens bore time. This will be explained hereafter and in particular with respect to the embodiment shown in Fig. 2.

According to an embodiment of the present invention, the safety device comprises:
- a retractable bore wall attachable to the mobile patient support, wherein the retractable bore wall is arranged to enclose the sedated patient during transport; or
- a retractable fence attachable to the mobile patient support, wherein the retractable fence is arranged around the mobile patient support.

This will be explained hereafter and in particular with respect to the embodiment shown in Fig. 2.

According to an embodiment of the present invention, the mobile patient support comprises an immersive audio-visual system for providing an interactive audio-visual environment to the sedated patient.

This will be explained hereafter and in particular with respect to the embodiment shown in Fig. 2.

According to an embodiment of the present invention, the controller is configured to generate the trigger signal based on information about a status of an imaging system.

For example, the scanner status may be in an unacceptable situation (such as, service mode, SW shutdown,), thus wake up process may be delayed or adapted.

According to an embodiment of the present invention, the trigger signal comprises at least one of:
- a trigger signal for the patient to receive an injection of a sedative mediation or a wake-up medication;
- a trigger signal for controlling the mobile patient support to enter or leave a bore;
- a trigger signal for retracting the retractable bore wall or for retracting the retractable fence;
- a trigger signal for controlling a content of the immerse audio-visual system; and
- a trigger signal for controlling one or more supporting devices to allow the sedated patient to stand up from the mobile patient support.

This will be explained hereafter and in particular with respect to the embodiment shown in Fig. 2.

According to an embodiment of the present invention, the wake-up management system further comprises a display configured to display current constraints for wake-up and/or predictive data for the wake-up procedure.

According to a third aspect of the present invention, there is provided a method of assessing a wake-up procedure of a sedated patient in an imaging process, comprising:
a) receiving patient profile data of the sedated patient and sedation data comprising a sedation state of the sedated patient;
b) applying a data-driven model to the patient profile data and sedation data of the sedated patient to estimate at least one timing in the wake-up procedure of the sedated patient;
   wherein the data-driven model has been trained on a training dataset obtained from historical data of one or more patients;
   wherein the training dataset comprises patient profile data and sedation data of the one or more patients as training inputs and at least one timing in the wake-up procedure of the one or more patients as training outputs; and
c) providing the at least one estimated timing.

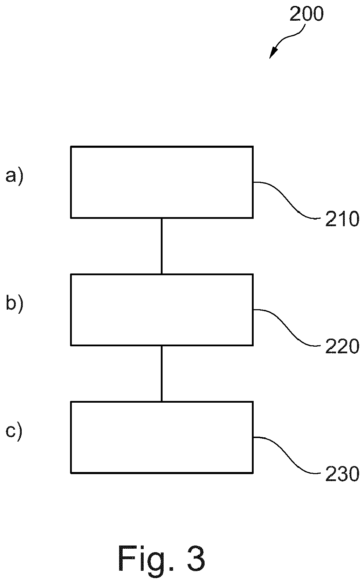

This will be explained hereafter and in particular with respect to the embodiment shown in Fig. 3.

According to another aspect of the present invention, there is provided computer program comprising instructions which, when the program is executed by a processor, cause the processor to carry out the steps of the method according to the third aspect and any associated example.

As used herein, the term "logic" and "module" may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality.

As used herein, the term "data-driven model" in the context of machine learning refers to a suitable algorithm that is learnt on the basis of appropriate training data. "Machine-learning" refers to the field of the computer sciences that studies the design of computer programs able to induce patterns, regularities, or rules from past experiences to develop an appropriate response to future data, or describe the data in some meaningful way. "Learning" in the context of machine learning refers to the identification and training of suitable algorithms to accomplish tasks of interest. In a machine learning algorithm, task performance improves measurably after having provided the data-driven model with more and more training data. The data-driven model is adapted based on the training data. The performance may be measured by objective test when feeding the trained data-driven model The performance may be defined by requiring a certain error rate to be achieved for the given test data. See T. M. Mitchell, "Machine Learning", page 2, section 1.1, McGraw-Hill, 1997.

The term "controller" is used generally to describe various apparatus relating to the operation of a stream probe apparatus, system, or method. A controller can be implemented in numerous ways (e.g., such as with dedicated hardware) to perform various functions discussed herein. A "processor" is one example of a controller which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).These and other aspects of the present invention will become apparent from and be elucidated with reference to the embodiments described hereinafter.

In various implementations, a processor or controller may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present disclosure discussed herein. The terms "program" or "computer program" are used herein in a generic sense to refer to any type of computer code (e.g., software or microcode) that can be employed to program one or more processors or controllers.

The term "user interface" as used herein refers to an interface between a human user or operator and one or more devices that enables communication between the user and the device(s). Examples of user interfaces that may be employed in various implementations of the present disclosure include, but are not limited to, switches, potentiometers, buttons, dials, sliders, track balls, display screens, various types of graphical user interfaces (GUIs), touch screens, microphones and other types of sensors that may receive some form of human-generated stimulus and generate a signal in response thereto.

The term "patient" may refer to human or animal.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically shows an apparatus for assessing a wake-up procedure, in accordance with an embodiment.
Fig. 2 schematically shows a wake-up management system.
Fig. 3 is a flowchart of a method of assessing a wake-up procedure, in accordance with an embodiment.
Fig. 4 is a flowchart of a wake-up management method, in accordance with an embodiment.

### DETAILED DESCRIPTION OF EMBODIMENTS

According to a first aspect of the present disclosure, there is provided an apparatus 10 for assessing a wake-up procedure of a sedated patient in an imaging process. The apparatus comprises an input unit 12, a processing unit 14, and an output unit 16.

The input unit 12 is configured to receive patient profile data and sedation data comprising information about a sedation state of the sedated patient.

The processing unit 14 is configured to apply a data-driven model to the patient profile data and sedation data of the sedated patient to *estimate* at least one timing in the wake-up procedure of the sedated patient. The data-driven model has been trained on a training dataset obtained from historical data of one or more patients.

The output unit 16 is configured to provide the at least one estimated timing.

Fig. 1 shows a schematic diagram of an apparatus 10 according to the first aspect of the present disclosure.

The input unit 12 is, in an example, implemented as an Ethernet interface, a USB (TM) interface, a wireless interface such as a WiFi (TM) or Bluetooth (TM) or any comparable data transfer interface enabling data transfer between input peripherals and the processing unit 14.

The input unit 12 is configured to receive patient profile data, which may include gender, age, Body Mass Index (BMI), cardiac fitness, etc. The patient profile data may be obtained from the exam metadata. The exam metadata may be obtained from the log file of a medical imaging apparatus, such as an X-ray imaging apparatus, or a magnetic resonance (MR) imaging apparatus. The exam metadata may also be obtained from connected information, data archiving systems, such as a radiological information system (RIS), a hospital information system (HIS), and/or a picture archiving and communication system (PACS), and/or from other workstations.

The input unit 12 is also configured to receive sedation data comprising information about a sedation state of the sedated patient. The sedation state may also be referred to as sedation depth or sedation level. An index value may be generated to represent the patient sedation state e.g. according to the American Society of Anesthesiologists (Standards, Guidelines and Statements, 2015):
"Minimal Sedation" (Anxiolysis) is a drug-induced state during which patients respond normally to verbal commands. Although cognitive function and coordination may be impaired, ventilatory and cardiovascular functions are unaffected;

"Moderate Sedation/Analgesia" (Conscious Sedation) is a drug-induced depression of consciousness during which patients respond purposefully to verbal commands, either alone or accompanied by light tactile stimulation. No interventions are required to maintain a patient airway, and spontaneous ventilation is adequate. Cardiovascular function is usually maintained;

"Deep Sedation/Analgesia" is a drug-induced depression of consciousness during which patients cannot be easily aroused but respond purposefully following repeated or painful stimulation. The ability to independently maintain ventilatory function may be impaired. Patients may require assistance in maintaining a patient airway, and spontaneous ventilation may be inadequate. Cardiovascular function is usually maintained; and

"General Anesthesia" is a drug-induced loss of consciousness during which patients are not arousable, even by painful stimulation. The ability to independently maintain ventilatory function is often impaired. Patients often require assistance in maintaining a patient airway, and positive pressure ventilation may be required because of depressed spontaneous ventilation or drug-induced depression of neuromuscular function. Cardiovascular function may be impaired.

The index value representative of the sedation state may be any measurable characteristic. The characteristic may be e.g. a continuous, or ordinal measurement. For example, because sedation is a continuum, the sedation state could have an index value on a continuous scale. As another example, the measurement of the sedation state may be on a decimal category ordinal scale from 1 (minimal sedation) to 3 (deep sedation).

Several methods may be used for determining a sedation state of the patient.

In an example, the sedation state may be estimated based on the sedation dose (optionally paired with the sedative medication) used for the patient. For patients who have been sedated (and optionally scanned) before, the sedation state may be estimated by comparing the sedation dose used for the present session with data recorded at previous occasions.

In another example, a sedation tracker may be provided for dynamically monitoring the sedation state of the sedated patient. For example, the input unit 12 may be configured to receive real-time measurement data indicative of a sedation state of the sedated patient from the sedation tracker. The processing unit 14 may be configured to continuously (e.g. every 30 seconds, 1 min, 5 min, or 10 min) adjust the at least one estimated timing and the at least one determined parameter according to the sedation level of the sedated patient. The sedation tracker provides real-time measurement data, which may help for precise timing prediction.

In an example of the sedation tracker, a remote haptic device may be used to mimic a very tactile nudge or a touch and/or a wearable skin trigger with heating and/or cooling to induce a pain sensation. The remote haptic device may be modulated to generate a specific physical form of nudge/tap at specific locations, i.e. impact regions, at cheeks, forehead, hand, etc. The impact region to generate the localized stimulus may be determined based on the assessment description of sedation as defined in various sedation determining scales. A range of remote haptic stimuli may be used, including, but not limited to, acoustic fields (e.g. ultrasound), electromagnetic fields (e.g. light), aerodynamic fields (e.g. air flow), magnetic fields, etc. Since they are bidirectional, they can also be used for closed loop monitoring to, assess the impact of nudging, its response and compare with expected sedation level awareness. Patient reactions in response to the haptic sensations may include, but are not limited to, a movement of a body part with the impact region, a change in a facial gesture of the patient, a change in a measured vital sign, a muscle response associated with the body part with the impact region, and a nerve response associated with the body part with the impact region. The movement of the body part and the change in the facial gesture may be detected with a camera or video based system. The vital sign may be monitored by a galvanic skin response (GSR) sensor. The muscle/nerve response may be detected by an electromyography (EMG) or electroencephalography (EEG) sensor. The sedation level may be determined by correlating the detected patient reaction for a haptic feedback with an anticipated response.

In another example of the sedation tracker, skin triggers with heating and/or cooling may be used. The measurement may be done with the help of an actuator device that triggers patient feedback in a well-defined way. The actuator could be a heating/cooling device, which at an exact defined position generates a defined input signal to the patient, thereby leading to a reaction of the patient that is correlated to the patient's sedation level.

In a further example of the sedation tracker, the imaging modality itself may be used to measure the response to suitable reflexes in order to determine the depth of sedation. For example, the pupil reflex in response to changes of illumination of the retina may be suitable for sedation depth measurement as detailed out below. The pupil reflex may be suitable for sedation depth monitoring in a magnetic resonance imaging (MRI) system. For example, the pupil reflex may be measured in an MRI system by repeated interleaving of dedicated iris MR imaging with the conventional scan protocol. As another example, the superficial reflexes have motor responses in response to scraping of the skin. Examples include the abdominal reflex, the cremaster reflex, the glabellar reflex and the normal plantar response. The latter involves flexing of the big toe upon stroking of the sole of the foot and may be particularly useful, because it involves gentle stimulation and a response with minor local motion. The glabellar reflex, also known as the "glabellar tap sign", involves eye blinking upon repetitive tapping on the forehead and seems similarly useful. The superficial reflexes may be suitable for sedation depth monitoring in an MRI system, in an X-ray imaging system, or in a computed tomography (CT) system. The MR imaging apparatus, the X-ray imaging apparatus, or the CT imaging apparatus may acquire a sequence of images of a body part to detect the response reaction, e.g., minor local motion, eye blinking, etc. As a further example, the withdrawal reflexes of limbs or fingers may be monitored because they can be easily stimulated and measured during diagnostic imaging if induced motion does not disturb imaging. The withdrawal reflexes may be suitable for sedation depth monitoring in an MRI system, in an X-ray imaging system, or in a CT system. These systems may acquire a sequence of images of a body part to detect the response reaction, i.e., the induced motion of limbs or fingers.

Optionally, the input unit 12 may receive calibration and training data from the first period of the imaging session to predict the second part, i.e. the wake-up procedure. In the present scan, the change in sedation history compared to the data from previous occasions may be used primarily to indicate wake-up. As an example, it may be considered that the wake-up time will scale with some power of the relative amount of sedative administered on the present and previous occasions and with some factor reducing/increasing the predicted wake-up if the administration moment of the sedative was earlier or later in the sedation process than the previous session.

The processing unit 14 may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality. Furthermore, such processing unit 14 may be connected to volatile or non-volatile storage, display interfaces, communication interfaces and the like as known to a person skilled in the art. A skilled person will appreciate that the implementation of the processing unit 14 is dependent on the compute intensity and latency requirements implied by the selection of signals used to represent positional information in a particular implementation.
The processing unit 14 is configured to apply a data-driven model to the patient profile data and sedation data of the sedated patient to estimate at least one timing in the wake-up procedure of the sedated patient.

For example, the data-driven model may be a neural-network (NN architecture). Broadly, the NN structure of the machine learning model includes a plurality of nodes, at least partly interconnected and arranged in different layers. The layers are arranged in one or more sequences. Each node is an entry capable of assuming a value and/or can produce an output based on input it receives from one or more nodes of an earlier layer. Each node is associated with a certain function, which may be a simple scalar value (node weight) but may also be with more complex linear or non-linear functions. A "connection" between nodes in two different layers means that the node in the later layer can receive an input from the node in the earlier layer. If there is no connection defined between two nodes, no output of one of the two nodes can be received by the other node as input. The node produces its output by applying its function to the input. This may be implemented as a multiplication of the received input by the scalar value (the node weight) of the node. The interconnected layered nodes with their weights, layer size, etc., thus forms a NN (neural network) model as an example of the data-driven model envisaged herein. The data-driven model may be stored in a matrix or tensor structure in a memory.

The data-driven model has been trained on a training dataset obtained from historical data of one or more patients. The training dataset comprises patient profile data and sedation data of the one or more patients as training inputs and at least one timing in the wake-up procedure of the one or more patients as training outputs. In other words, the sedation data of one or more patients are paired with additional patient-specific parameter vectors (such as height, weight, age, gender, BMI, etc.) as input, and the network parameters are optimized to infer the at least one timing (such as wake-up time, time to administer medication, bore-exit, stand-up time, etc.) in the wake-up procedure as output. When training the data-driven model, the additional patient-specific parameter vectors are used to calibrate the effect of certain dose of sedation and/or certain sedative medication on a particular person.

In an example, the training dataset may be obtained from historical data of a plurality of other patients. For example, the data-driven model may be trained on various population data of their responses to the sedation. The various population data may be obtained from connected information, data archiving systems, such as a radiological information system (RIS), a hospital information system (HIS), and/or a picture archiving and communication system (PACS), and/or from other workstations.

In an example, for patients who have been sedated (and optionally scanned) before, the training dataset may comprise previous data of the same patient, i.e. the sedation history and various timings (e.g. wake-up time, time to administer medication, bore-exit, stand-up time, etc.) recorded at previous occasions. Optionally, the training dataset may also comprise population data. The training dataset may be biased towards the previous data of the same patients. For example, the previous data of the same patients may be provided more weights compared to the population data. In this way, the trained data-driven model is more patient-specific and therefore the prediction of the timing (wake-up time, time to administer medication, bore-time, stand-up time, etc.) may be more accurate for a particular patient. Once trained, this structure forms the trained data-driven model, which can be held on one or more memories. The trained data-driven model can thus be used to predict at least one timing in the wake-up procedure.

In an example, the data-driven model may be used to estimate the time left for the patient to come out of the sedation (i.e. wake-up time). For example, the data-driven model may estimate the time left for the patient to come out of "Deep Sedation/Analgesia" and enter "Moderate Sedation/Analgesia" (Conscious Sedation), during which patients respond purposefully to verbal commands, either alone or accompanied by light tactile stimulation.

In an example, the time is estimated when the patient can leave the scanner in a safe mode (i.e. stand-up time). For example, the data-driven model may estimate the time for the patient to have a sedation state below a critical value.

The apparatus 10 may allow a precise timing prediction for a defined wake-up procedure. The timing prediction may help in understanding when the patient will wake-up from the sedation and hence next steps in the workflow, such as post imaging as exiting the bore, and/or performing autonomous standing up from the patient support. The timing prediction may thus enable a much better planning for the room and the imaging process with well adapted processes for the patient according to his individual profile. Besides higher acceptance the apparatus may also reduce the risks, as the wake-up process is well planned based on the timing prediction, thereby improving the overall quality and patient experience.

Optionally, the processing unit 14 may be configured to determine a sequence of workflow steps in the imaging process and/or an imaging protocol based on the at least one estimated timing. With the timing information in the wake-up procedure, the different process steps may be brought into the best matching sequence in the workflow. The workflow may be optimized timewise, but also with optimized patient experience and minimized risk. Based on the timing prediction, the workflow steps may be optimized based on a prescribed scan procedure, such as speeding the scan procedures, increasing the sedation dose, etc. For example, depending on the individual patient profile, sound, music, light, special smelling, and the like may be used to increase the speed of the wake-up procedure. Additionally, the processing unit 14 may determine how and when the patient comes from the sedated level to a level when he can do next steps on his own, and finally leave the scanning room on his own.

Optionally, the processing unit 14 may be configured to determine the delivery of required support for the sedated patient and/or staff support. The required support for the patient may include, but are not limited to, audio information, video guidance, transport support (e.g. wheelchair or automatic wheelchair, etc). One example of the staff support is the injection of sedation medicine (the decrease of dose) and also the counterpart to use a kind of "wake up medication" supporting the natural human process - also some emergency wake up medication might be used. This is a medication that could be given in critical situation that could be detected during the continuous monitoring procedures. Even several medication slots that could be selected may be predicted. A switch from "sedation" to "wake up" with different dose levels may allow individual patient treatment from entering the scanner during the complete scanning process until full wake up.

With this estimation, the required support for the sedated patient and/or staff support may be estimated and delivered in time.

The output unit 16 is, in an example, implemented as an Ethernet interface, a USB (TM) interface, a wireless interface such as a WiFi (TM) or Bluetooth (TM) or any comparable data transfer interface enabling data transfer between output peripherals and the processing unit 14.

The output unit 16 is configured to provide the at least one estimated timing and optionally the at least one determined parameter (such as sequence of workflow steps, imaging protocol, delivery of patient support, delivery of staff support) e.g. to a display device and/or to a controller for device management according to the timing/workflow prediction.

According to a second aspect of the present disclosure, there is provided a wake-up management system 100. The wake-up management system comprises:
- an apparatus 10 according to the first aspect of the present disclosure and any associated example; and
- a controller 20 configured to generate a trigger signal for the sedated patient and/or one or more devices to perform an action based on the at least one estimated timing in the wake-up procedure.

Fig. 2 shows schematically and exemplarily an embodiment of a wake-up management system 100 according to the second aspect of the present disclosure. The wake-up system 100 comprises an apparatus according to the first aspect of the present disclosure and any associated example. The wake-up system 100 also comprises a controller 20 configured to generate a trigger signal for the sedated patient and/or one or more devices to perform an action based on the at least one estimated timing in the wake-up procedure. Optionally, the controller may be configured to generate the trigger signal based on information about a status of an imaging system. For example, if the scanner status is in an unacceptable situation (e.g. service mode), the controller may be configured to generate a trigger signal for the sedated patient and/or one or more devices to delay or adapt the wake-up process.

Moreover, although shown as in a single device (e.g. desktop computer) in Fig. 2, the apparatus 10 and the controller 20 may be implemented as separated devices. For example, the apparatus 10 may be a desktop computer, laptop computer, or other mobile device, whilst the controller 20 may be implemented as a combination of dedicated hardware (e.g. FPGAs) to perform the controlling functions.

The wake-up system 100 may be used to optimize radiology workflow in a medical imaging system 110, such as magnetic resonance imaging (MRI), MR LINAC, positron emission tomography-magnetic resonance (PET-MR), MR-hybrid, etc.

The most simple solution to de-risk the unsupervised wake-up of the patient would be to leave the patient in the imaging system in this phase until the patient is ready to be transported autonomously. However, while the above artificial intelligence (AI)-based timing prediction apparatus 10 may predict and shorten this wake-up time, still, bore time will be longer than for a fully supervised wake-up, because here typically staff takes the patient out of the bore irrespective of sedation. Moving a sedated patient out of the bore autonomously on a self-driving patient support may confuse the patient considerably and induce the risk of anxiety or even falling down from the support. The following examples may provide means for a safe and undisturbed unsupervised exit of the patient from the bore under sedation. Similarly, the following examples may also enable to induce sedation before the patient enters the imaging system, which again shortens bore time.

In an example, the one or more devices controlled by the controller may include a mobile patient support 120. The mobile patient support 120 as used herein may refer to an apparatus, such as a bed shown in Fig. 2, a wheelchair, or an autonomous movement unit, for transferring a patient from one location to another within a healthcare facility. The patient transfer apparatus may be provided with a patient support system, such as connected monitors, sensors, residual devices strapped, e.g. drugs, saline, etc.

In order to reduce the risk of falling down from the support, the mobile patient support 120 may comprise a safety device 130 for preventing the sedated patient from falling down from the mobile patient support during transport.

For example, the safety device 130 may comprise a retractable bore wall attachable to the mobile patient support. The retractable bore wall may be arranged to enclose the sedated patient during transport. This example is illustrated in Fig. 2. In this example, an extra bore wall attached to the mobile patient support 120 is proposed. The retractable bore wall may enclose the patient during transport to and from the imaging system and may serve two purposes. Firstly, the view of the patient is blocked to avoid anxiety or disturbance, and secondly, it prevents the patient from falling down from the patient support during transport. The bore wall may be designed such that it can be retracted into the patient support to release the patient e.g. according to a trigger signal provided by the controller 20. It may be designed as a rigid plastic sheet or in a light-weight fashion similar to a removable sun blind on rails.

For example, the safety device 130 may comprise a retractable fence attachable to the mobile patient support, wherein the retractable fence is arranged around the mobile patient support. Alternatively to the retractable bore wall as mentioned above, a retractable fence around the mobile patient support is proposed that basically serves the same purpose as the mobile bore wall but is less spacious and may provide better patient access for staff. Height of the fence may be e.g. in the order of 20 cm.

Optionally, the mobile patient support 120 may comprise an half or full immersive audio-visual system for providing an interactive audio-visual environment to the sedated patient. The interactive audio-visual environment may serve three purposes. Firstly, the interactive audio-visual environment may provide the required support for the sedated patient, such as audio information and/or video guidance, to the patient to reduce the confusion of the patient and thus the risk of anxiety of the patient, e.g. when the sedated patient is moved out of the bore autonomously on a self-driving patient support. Secondly, the interactive audio-visual environment may provide e.g. sound, music, and/or light to increase the wake-up speed to bring wake-up procedure into alignment with the estimated timing. Thirdly, a half or fully immersive audio-visual system integrated with the mobile patient support may serve the same purpose as the mobile bore wall, but is less spacious and provides better patient access for staff.

The controller 20 may trigger signals in response to the timing prediction for the patient to receive sedation dose, enter a bore 112 (see Fig. 2) of the medical imaging system 100, leave the bore 112, retract the extra bore wall/fence of the mobile patient support, control the content of the patient entertainment system, and to allow the patient to stand up from the mobile patient support. The trigger signals may be sent to a display for a remote operator and/or a controller for controlling the device to perform an action.

In an example, the trigger signal may comprise a trigger signal for the patient to receive an injection of a sedative medication or a wake-up medication. The trigger signal may be sent to a display for a staff to delivery staff support. A switch from "sedation" to "wake up" with different dose levels may allow individual patient treatment from entering the scanner during the complete scanning process until full wake up. This may allow better matching trigger for imaging during the complete sequence as it is synchronized with the medication.

In an example, the trigger signal may comprise a trigger signal for controlling the mobile patient support to enter or leave a bore. Optionally, a trigger signal for retracting the retractable bore wall or for retracting the retractable fence may be provided. Optionally, a trigger signal for controlling a content of the immerse audio-visual system may be provided. Accordingly, bore time will be shorter than a fully supervised wake-up, because the patient may be moved out of the bore irrespective of sedation. Moving a sedated patient out of the bore autonomously on a self-driving patient support may confuse the patient considerably and induce the risk of anxiety or even falling down from the support. The retractable bore wall or the retractable fence provide means for a safe and undisturbed unsupervised exit of the patient from the bore under sedation. The trigger signal may cause the retractable bore wall or the retractable fence to retract once it is determined that the sedation level is below a critical value (e.g. a value indicates that cognitive function and coordination are unaffected). Similarly, the trigger signal may control a content of the half or fully immerse audio-visual system to provide audio information and/or video guidance to reduce the anxiety of the patient.

I n an example, the trigger signal may comprise a trigger signal for controlling one or more supporting devices to allow the sedated patient to stand up from the mobile patient support. For example, when the patient is located at the patient trolley/bed, the patient may be fixed/locked by holders, such as radiation therapy holders and masks, or may be embedded with coils or flexible mattress supports. Additionally, sensors and/or actuators may be placed on the patient and need to be deactivated before the wake up process. The trigger signal may be used to control one or more of these supporting device. For example, a trigger signal may be sent via physical cables or wirelessly to deactivate the sensors and/or actuators before the wake up process.

Optionally, a display (not shown) may be provided, which is configured to display current constraints for wake-up and/or predictive data for the wake-up procedure e.g. for a remote or nearby operator and/or a decision control software. The current constraints to be display may include e.g. information about supporting device (e.g. coils, masks, holders, fixation, etc.), tactile sensors (e.g. EM sensors, camera, impedance, etc.), and information about the status of the scanner. The predictive data for the wake-up procedure may include various timings (e.g. wake-up time, time to administer medication, bore-exit time, stand-up time, etc.) and further workflow-related information (e.g. sequence of workflow steps, imaging protocol, delivery of required support for the sedated patient, delivery of staff support, etc.).

For example, to display the wake up process, a flexible portable display in form as a curtain around the patient may be located. The curtain is an interactive flexible display, which shows up information (e.g. patient profile and sedation status) about the patient and information the patient and information (e.g. dynamic light, ambient light) about the wake up process. The display may be portable or in combination with the mobile patient support 120 and wirelessly linked to the apparatus 10 and the controller 20 for controlling the wake up process.

According to a third aspect of the present disclosure, there is provided a method 200 of assessing a wake-up procedure of a sedated patient in an imaging process, comprising:
a) receiving 200 patient profile data of the sedated patient and sedation data comprising a sedation state of the sedated patient;
b) applying 220 a data-driven model to the patient profile data and sedation data of the sedated patient to estimate at least one timing in the wake-up procedure of the sedated patient, the data-driven model has been trained on a training dataset obtained from historical data of one or more patients; and
c) providing 230 the at least one estimated timing.

Reference is now made to the flowchart in Fig. 3 to explain in more detail a method according to the third aspect of the present disclosure. The method may be understood to underline operation of the above mentioned apparatus 10 for assessing a wake-up procedure of a sedated patient in an imaging process. However, it will be also understood that the method steps explained in Fig. 3 are not necessarily tied to the architecture of the apparatus 10 as described above in relation to Figs. 1 and 2. More particularly, the method described below may be understood as teachings in their own right. The flow chart of Fig. 3 will be explained assuming that the data-driven model has been sufficiently trained, for instant as explained above in Fig. 1.

In step 210, i.e. step a), patient profile data of the sedated patient is received e.g. from exam metadata. The patient profile data may comprise gender, age, BMI, fitness level of the sedated patient. In addition, sedation data comprising a sedation state of the sedated patient may be received, e.g. from a sedation tracker as explained above in Fig. 1. Optionally, the sedation data may comprise a sedative medication and/or sedation dose used for the sedated patient.

In step 220, i.e. step b), a data-driven model, such as neural network explained above, is applied to the patient profile data and sedation data of the sedated patient to estimate at least one timing in the wake-up procedure of the sedated patient. The data-driven model has been trained on a training dataset obtained from historical data of one or more patients. The historical data may comprise population data obtained from connected information, data archiving systems, such as a radiological information system (RIS), a hospital information system (HIS), and/or a picture archiving and communication system (PACS), and/or from other workstations. The historical data may comprise data of the same patient recorded at previous occasions, if the patient has a sedation history.

Optionally, step 220 may further comprise the step of determining at least one of the following parameters based on the at least one estimated timing: sequence of workflow steps in the imaging process, imaging protocol, delivery of required support for the sedated patient, and delivery of staff support. These parameters may provide workflow-related information. This may allow the pre-defined process to be adaptively adjusted with the individual processed timing elements to customize the workflow.

In step 230, i.e. step c), the at least one timing is provided e.g. for a remote or nearby operator and/or a decision control software.

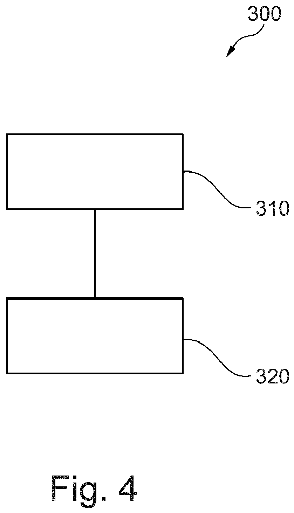

Referring now to the flow chart in Fig. 4, this shows a method 300 for wake-up management, in particular as explained above in Fig. 2. The method may be understood to underline operation of the above mentioned wake-up system 100. However, it will be also understood that the method steps explained in Fig. 4 are not necessarily tied to the architecture of the wake-up system as described above in relation to Fig. 2. More particularly, the method described below may be understood as teachings in their own right.

In step 310, a wake-up procedure is accessed. The wake-up procedure may include timing prediction and workflow estimation as described above in Figs. 1 and 3.

In step 320, a trigger signal is generated for the sedated patient and/or one or more devices to perform an action based on the at least one estimated timing in the wake-up procedure. The trigger signal may be sent to a display and/or to a controller for controlling the one or more devices to perform an action.

As explained above with reference to Fig. 2, the trigger signal may comprise trigger signal comprises one or more of a trigger signal for the patient to receive an injection of a sedative mediation or a wake-up medication, a trigger signal for controlling the mobile patient support to enter or leave a bore, a trigger signal for retracting the retractable bore wall or for retracting the retractable fence, a trigger signal for controlling a content of the immerse audio-visual system, and a trigger signal for controlling one or more supporting devices to allow the sedated patient to stand up from the mobile patient support.

In other words, the proposed method may trigger signals for the patient to receive sedation dose, enter the bore, leave the bore, retract the extra bore wall/fence of the mobile patient support, control the content of the patient entertainment system, and/or allow the patient to stand up from the patient support. In this way, the workflow is optimized timewise but also with optimized patient experience and minimized risk. The risk of any uncontrolled action by the patient may be minimized with the extra bore wall/fence of the mobile patient support and/or the patient entertainment system, so that there is less or no risk even in case there is no staff available directly or within a short period of time. This may also allow to reduce the bore-time, because the patient may be moved out of the bore irrespective of sedation (thanks to the extra bore wall/fence and/or the patient entertainment system).

It should also be understood that, unless clearly indicated to the contrary, in any methods claimed herein that include more than one step or act, the order of the steps or acts of the method is not necessarily limited to the order in which the steps or acts of the method are recited.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one. "

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. An apparatus (10) for assessing a wake-up procedure of a sedated patient in an imaging process, comprising:
- an input unit (12);
- a processing unit (14); and
- an output unit (16);
wherein the input unit is configured to receive patient profile data and sedation data comprising information about a sedation state of the sedated patient;
wherein the processing unit is configured to apply a data-driven model to the patient profile data and sedation data of the sedated patient to estimate at least one timing in the wake-up procedure of the sedated patient, wherein the data-driven model has been trained on a training dataset obtained from historical data of one or more patients; and
wherein the output unit is configured to provide the at least one estimated timing.

2. Apparatus according to claim 1,
wherein the processing unit is configured to determine at least one of the following parameters based on the at least one estimated timing:
- sequence of workflow steps in the imaging process;
- imaging protocol;
- delivery of required support for the sedated patient; and
- delivery of staff support; and
wherein the output unit is configured to provide at least one determined parameter.

3. Apparatus according to claim 1 or 2,
wherein the sedation data further comprises a sedative medication and/or sedation dose used for the sedated patient.

4. Apparatus according to any one of the preceding claims,
wherein the training dataset is obtained from historical data of the sedated patient and/or from historical data of a plurality of other patients.

5. Apparatus according to any one of the preceding claims,
wherein the at least one timing in the wake-up procedure comprises one or more of:
- wake-up time;
- time to administer medication;
- bore-exit time; and
- stand-up time.

6. Apparatus according to any one of the preceding claims,
wherein the input unit is further configured to receive real-time measurement data indicative of a sedation state of the sedated patient;
wherein the processing unit is configured to continuously adjust the at least one estimated timing and the at least one determined parameter according to the sedation level of the sedated patient.

7. A wake-up management system (100), comprising:
- an apparatus (10) according to any one of the preceding claims; and
- a controller (20) configured to generate a trigger signal for the sedated patient and/or one or more devices to perform an action based on the at least one estimated timing in the wake-up procedure.

8. Wake-up management system according to claim 7,
wherein the one or more devices comprise:
- a mobile patient (120) support for transferring a patient to and from a medical imaging system;
wherein the mobile patient support comprises a safety device (130) for preventing the sedated patient from falling down from the mobile patient support during transport.

9. Wake-up management system according to claim 8,
wherein the safety device comprises:
- a retractable bore wall attachable to the mobile patient support, wherein the retractable bore wall is arranged to enclose the sedated patient during transport; or
- a retractable fence attachable to the mobile patient support, wherein the retractable fence is arranged around the mobile patient support.

10. Wake-up management system according to claim 8 or 9,
wherein the mobile patient support comprises:
- an immersive audio-visual system for providing an interactive audio-visual environment to the sedated patient.

11. Wake-up management system according to any one of claims 7 to 10,
wherein the controller is configured to generate the trigger signal based on information about a status of an imaging system.

12. Wake-up management system according to any one of claims 7 to 11,
wherein trigger signal comprises at least one of:
- a trigger signal for the patient to receive an injection of a sedative mediation or a wake-up medication;
- a trigger signal for controlling the mobile patient support to enter or leave a bore;
- a trigger signal for retracting the retractable bore wall or for retracting the retractable fence;
- a trigger signal for controlling a content of the immerse audio-visual system; and
- a trigger signal for controlling one or more supporting devices to allow the sedated patient to stand up from the mobile patient support.

13. Wake-up management system according to any one of the preceding claims, further comprising:
- a display configured to display current constraints for wake-up and/or predictive data for the wake-up procedure.

14. A method of assessing a wake-up procedure of a sedated patient in an imaging process, comprising:
a) receiving (210) patient profile data of the sedated patient and sedation data comprising a sedation state of the sedated patient;
b) applying (220) a data-driven model to the patient profile data and sedation data of the sedated patient to estimate at least one timing in the wake-up procedure of the sedated patient, wherein the data-driven model has been trained on a training dataset obtained from historical data of one or more patients; and
c) providing (230) the at least one estimated timing.

15. A computer program element comprising instructions which, when the program is executed by a processor, cause the processor to carry out the steps of the method of claim 14.
